# EUROPEAN PATENT APPLICATION

(11) **EP 4 776 298 A1**
(43) Date of publication of application: **15.07.2026**
(21) Application number: 25150651.5
(22) Date of filing: 08.01.2025
(51) Int. Cl.: G16H 20/40, G16H 30/40, G16H 40/63, G16H 50/20

(54) **NEURAL NETWORK SELECTION FOR DEVICE TRACKING**

(71) Applicant: Koninklijke Philips N.V., 5656 AE Eindhoven (NL)
(72) Inventor: HAASE, Hans Christian, Eindhoven (NL); GRASS, Michael, 5656AG Eindhoven (NL)
(74) Representative: Philips Intellectual Property & Standards

(57) **Abstract**

A system (SS) and related method for supporting procedure in respect of entity (PAT). The procedure is guidable based on a stream of frames (*mj*) acquirable by an interventional imaging apparatus (IA) of the entity (PAT). The system comprises an input interface (IN) for receiving input data (d) indicative of an intended one implements (Pi, P1,P2) in a current FOV as per a current frame *(mt0*) displayable by a display device (DID). A selector module (SM) selects, based on the input data, a trained machine learning model. The selected ML model (M*) supports the procedure by being capable of providing a detection response, based on a current frame, of a part (Pij) of the intended implement as per the current frame (*mt0*)*,* and in at least one future frame (*mt*'). Output interface (OUT) provides output data (e) including feedback data on the selected model.

## Description

### FIELD OF THE INVENTION

The invention relates to a system for supporting a procedure in respect of an entity, to a related method, to a related arrangement, to a computer program element, and to a computer readable medium.

### BACKGROUND OF THE INVENTION

An IGP ("Image guided procedure"), such as in IR ("international radiology"), may be used to treat and/or diagnose, for example, stroke, thrombosis and similar ailments that afflict large parts of the population, particularly the elderly. Common to such types of conditions is an obstruction in blood vessels by a mass of material, such as blood clot or other. X-ray based imaging is an imaging technique that allows acquiring imagery of the internal organs or anatomies of a patient. Thus, the obstruction may be located in the afflicted blood vessel by X-ray based imaging, such as in angiography or fluoroscopy, or similar modalities. In addition, such obstructions may be removed by using specific implements, such as an aspirator device, that can be placed inside the afflicted vessel. Suction may be applied by a suction implement to assist clot removal or removal of other material that may cause health problems.

In such or other IGPs, computational models, such as machine learning models (e.g., neural networks) can be trained to support various tasks in such IGPs. Detecting, such as tracking, medical devices used in such IGPs, such as tools or implements, is a consideration as this enables a broad range of applications like robotic feedback loops, device enhancement or automatic system actions (e.g. controlling collimators, zooming, panning, etc.). Since there are many different kinds of devices or implements that may be used, sometimes simultaneously, sometimes sequentially, it may not be feasible, even for an ML model, to work put which device or implement to track at any given moment.

Additionally, some ML models (e.g., neural networks) may be trained on a specific device, a specific portion of a device (e.g. tip recognition) or a specific set of devices. On the other hand, more generalist ML models that may be capable of detecting all or a large class of devices, may not be practical since, depending on the procedure, its phase, user preference, etc. or the context, not all devices should be tracked, enhanced, or used for supporting actions. In addition, the processing power that is needed to track implements that are currently not relevant, or not even visible, should be saved for the relevant tasks.

As an example: In a first phase of an IGP, a full metal 0.036" guidewire tip must be tracked for automated collimation. In the second phase, the tip of a 0.014 guidewire with a 5 cm radio opaque tip must be tracked for automated collimation. In the last phase, the radio opaque markers of a Phenox flow diverter must be tracked for automated collimation. In the last phase, the radio opaque tip of the 0.014 guidewire is still visible (that is, is still in the FOV), but should be ignored by the tracking as it is irrelevant at this time. Also, previously placed devices such as a flow diverter ought to be ignored by the tracking since it is already deployed, etc.

### SUMMARY OF THE INVENTION

There may therefore be a need in image guided procedures for efficient use of computation models. The proposed set-up provides a robust high performance low latency environment/solution efficient use of computational models in performing detection, in particular tracking tasks of structures of interest across a stream of frames. It can be used for efficient support of image guided procedures of whichever type.

An object of the present invention is achieved by the subject matter of the independent claims where further embodiments are incorporated in the dependent claims. It should be noted that the following described aspects of the invention equally apply to the related method, to the imaging arrangement, to the computer program element and to the computer readable medium.

According to one aspect there is provided a system for supporting a procedure in respect of an entity, the procedure guidable based on a stream of frames acquirable by an interventional imaging apparatus of the entity, in respect of which the procedure is performable via one or plural implements, at least one of which is residable, at least at times, in a field of view (FOV), of the imaging apparatus , the system comprising:
an input interface for receiving, via a user interface , input data indicative of an intended one of the implements in a current FOV as per a current frame displayable by a display device;
a selector module operable to select, based on the input data, a trained machine learning ("ML") model from a set of trained ML models, the selected ML model capable of supporting the procedure by being capable of providing a detection response, based on a current frames, of at least a part of the at least one of the intended implements as per at least the current frame, and/or in at least one future frame; and
an output interface for providing output data including feedback data to a user on the selected model based on the said response.

In embodiments, the input data is explicitly or implicitly indicative of an in-image location in respect of the intended one of the implements. More specifically, in some embodiments, ii) the input data is indicative of a task, the procedure, phase of the procedure, or sub-procedure, to be performed, and/or wherein ii) the input data is indicative of an in-frame location of an image structure (e.g., projection footprint due to radiation opacity of the implement(s)) of the intended one of the implements, and wherein the said task, procedure, phase of the procedure, or sub-procedure is supportable by one or more of the implements.

In embodiments, the user interface is a graphical user interface.

In embodiments, the selection operation by selector module is dynamic so as to change with FOV.

In embodiments, the selection operation by selector module of the machine learning, ML, model from the set of ML models causes (automatic) deselection of other one or more models of the set selected earlier.

In embodiments, the selection operation by selector module is further based on background as per the FOV.

In embodiments, the selection operation by selector module is based on responses elicitable from the models in the set, based on the said input.

In embodiments, the selector module is capable to so select based on evaluating a metric configured to measure respective responses of models from the set of said models.

In embodiments, the selector module, in selecting the model, capable to disregard a candidate model from the said set that failed a selection test, and to further disregard all other such models that are similar the candidate model.

In embodiments, the output data includes evidential information capable of indicating evidence on the appropriateness of the selection in supporting a task.

In embodiments, the said task includes causing controlling of equipment.

In embodiments, the said equipment includes any one of more of: the imaging apparatus or a part thereof, a robot, and/or other one or more items of equipment usable to support the task.

In embodiments, the equipment includes a collimator of the imaging apparatus.

In embodiments, the feedback includes a visual modulation of a visualization of the current as displayed by the display device.

In embodiments, the feedback includes providing information on the training of the selected model.

In another aspect there is provided an arrangement for image guided support of a procedure, comprising the system of any one of the previous embodiments or aspect, and further comprising any one or more of: i) the imaging apparatus, ii) display device on which the frames are displayable, iii) at least one data storage or memory on which is storable at least one of the models that form the said set, iv) at least one of the implements, (v) at least one of the controllable equipment.

In another aspect there is provided a computing implemented method for supporting a procedure in respect of an entity, the procedure guidable based on a stream of frames acquirable by an interventional imaging apparatus of the entity, in respect of which the procedure is performable via one or plural implements, at least one of which is residable, at least at times, in a field of view, FOV, of the imaging apparatus , the method comprising :-
receiving, via a user interface , input data indicative of an intended one of the implements in a current FOV as per a current frame displayable by a display device;
selecting, based on the input data, a trained machine learning model from a set of trained ML models, the selected ML model capable of supporting the procedure by being capable of providing a detection response, based on a current frames, of at least a part of the at least one of the intended implements as per at least the current frame, and in at least one future frame; and
providing output data including feedback data to a user on the selected model based on the said response.

In another aspect there is provided a computer program element, which, when being executed by at least one (data) processing system (ag, a computing system), is adapted to cause the processing unit to perform the method.

In another aspect there is provided at least one computer readable medium having stored thereon the program element.

In another aspect there is provided method of training in the models in the set of use in the system or method as per of any one or more of the above mentioned aspects and embodiments.

The proposed system and method are based on said set of computational models, such as trained machine learning models (e.g., neural networks) for segmentation and/or tracking of devices and/or implements. Operation is based on user input (e.g. a click, implement selection, etc.), and a selection policy (e.g., based on a metric) that defines which model (target model) fulfills (e.g., best fulfills) the need that is indicated by the user input. Feedback on selection may be provided to user that provides information of the model selection.

The proposed setup allows managing for a user a possibly large set of trained ML models (such as, neural networks) , wherein each model is trained on a specific, different, task. The proposed system allows at least semi-automated selection of a target model from the set, thereby identifying a suitable (e.g. best) model or a specific task that is required for supporting a procedure. A computing-assisted selection method is provided, some embodiments including user interaction and user feedback.

The proposed set-up is particularly suited for in-image guided procedures where a number of types and category of input implements may be used, possibly multiple ones at the same time in different states, shapes or where different parts of implement(s) is used. Thus, this work situation may define different locations of interest that need to be independently tracked and such tracking needs or detection needs are changing dynamically during the procedure as the procedure proceeds along or through different phases and sub-procedures.

Instead of using one or few generalist computational models that may be capable of detecting a variety of such tools by way of their representative image structure, herein, instead, a different approach is used where a pool of specialist models are harnessed efficiently. These specialist models have been individually trained to in-frame/image detect (that is, to detect structures within a given image/frame) respective, possibly a quite narrow, category, class type of implements. More generalist models may been found to consume considerable computing power, as opposed to the more modest energy consumption of such specialist models, which can also operate more efficiently, more accurately, more robustly in their respective domain. Also, generalist models may expand computing resources on detecting devices not relevant at a given time in the IGP. The proposed setup is therefore of particular use for implementation, at least in parts, in mobile computing devices (laptops, smartphones, tablets, etc.), for example in form of an "app" and/or in any other form, such as cloud based or other.

The facilitator, on input from user, may dynamically de-select the currently selected model during the procedure thus, affording very efficient handling of such models. Based on output from the selected models, such as in-frame localization/detection output, other supportive equipment can be controlled that may require precise location of the respective implement or part thereof of interest at a given time in the procedure. Strain and fatigue on user can be reduced, which can be an asset in particular stress prone environments such as in medical clinics, trauma labs, "cath labs", etc.

The model's output data (e.g., a device detection or tracking result) may be provided by model during selection by the selection module, or thereafter, once selected. During selection or after selection, the model's output may be visualized, such as to form feedback data, The feedback data so visualized may provide evidentiary information to user that selection was apt and proper. After selection, the model's output may be optionally still visualized across frames. In either case, as an example, whilst the selected model is operable to detect and/or track the relevant implement's location, this location may be output in terms of a bounding box for example, a cross-hair widget for a single co-ordinate, or a group of co-ordinates, or any other.

After model selection, instead of or in addition to so visualizing, the output data, in particular tracking information, is provided as control data to control circuitry for an applicable equipment that is to support the image guided procedure. Such control circuitry may be operable to translate tracking information into control commands for an applicable piece of equipment. For example, such equipment may include a collimator of the imaging apparatus. For example, the tracking data may be used to control collimator to collimate, and possibly re-collimate, dynamically, into respective collimation windows focused on the implement as detected by the selected model.

Other such control operation and related equipment may include changing other imaging geometry, such as moving imaging apparatus' patient table, and/or moving its X-ray source and/or its detector for better imaging performance and provision of FOV for the user that is apt for the current procedure phase or its sub-procedure. In other embodiments, a robot is controlled based on the tracking information (detected location of implement) to support user or to autonomously perform at least part of the procedure.

The model (also referred to herein as a "target model" (there may be more than one), once selected, and if a different phase of procedure or sub-procedure is entered into, the user may wish a new model to be selected to better support the next step phase or sub-procedure. Thus, the user may indicate in any one of the follow up frame, new input data in relation to a new location pertaining to new implement or part, or different part of current implement, upon which the selector module selects a new model, and so forth. Preferably, upon selection of new model, the earlier one or more models are de-selected, and no longer used. This ensures specialist response at each phase or sub-procedure of the procedure. However, as another option, also envisaged, multiple models may remain "active" to perform detection for different implements or their parts, as needed.

The indicated in-frame location (the terms, in-image and in-frame will be used interchangeably herein) may implicitly define task, a phase of sub-procedure of the image guided procedure. For example, if user wishes to have one task performed based on the tracking of one implement, and another task may be performed based on the tracking of another implement. In some cases, the mere indication of a location in current frame may be sufficient to indicate the task, procedure's phase, sub-procedure, etc., but in other cases user may further provide additional context data so facilitators system can resolve this dual-channel information into the user intended task, for better, apt, model selection.

For example, the user could specify the task together with the location of the device. E.g. Task collimation, track device A at location X, Task zoom image, track device B at location Y. The same with procedure stage. The user may indicate e.g. "Navigation" or "Catheter exchange". Based on this information the tracking need would change. E.g. Full guide wire during navigation, only tip during catheter exchange. The task could then also change, for example, from automated collimation to automated warning if device positioning changes unintentionally, etc.

The proposed method and system may be said to be based on said set of ML models (e.g., neural networks), trained on clinical images to recognize, segment, and/or track various types of devices/implements. A subset of ML models may be trained to indicate only a particular part of a device, e.g. the tip, a marker, or a sensor on the device.

The proposed system and method may be used for vascular any IR /IG(image guided) procedures, or for other clinical applications, such as pulmonary embolism, coronary thrombus, and others.

The term *"user"* relates to a person, such as medical personnel or other, operating the imaging apparatus or overseeing the imaging procedure. In other words, the user is in general not the patient.

As used herein, the term "implement" may refer to any kind of tool, device, or instrument used during an image guided procedure or treatment.

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of an imaging arrangement;
Fig. 2 illustrates an image guided operation based on one or more implements and their in-image representation;
Fig. 3 shows a timeline of such image guided procedure, and exemplary frames acquired during such a procedure;
Fig. 4 shows a schematic block diagram of a facilitator system as may be used in facilitation of an image guided procedure; and
Fig. 5 shows a schematic flow chart of a computer-implemented method of facilitating an image guided procedure.

### DETAILED DESCRIPTION OF EMBODIMENTS

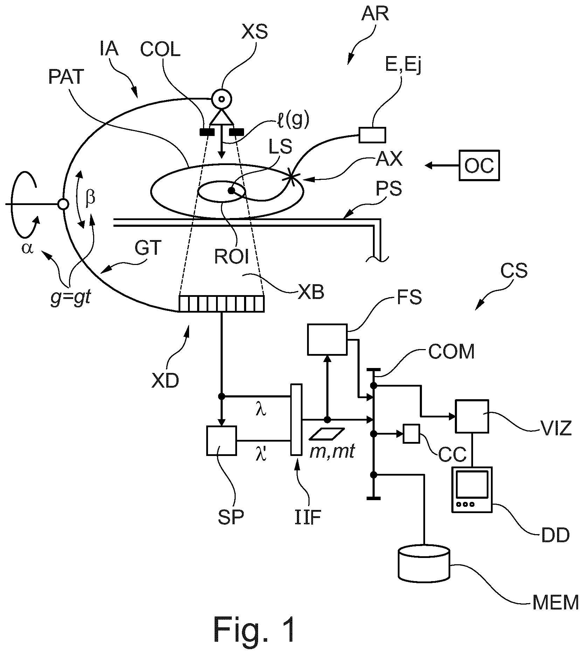

Turning first to Fig. 1, this shows a block diagram of a, in embodiments, medical imaging arrangement MIA. The imaging arrangement AR is configured to facilitate image-guided procedures, Such procedures may involve use of one or more plural implements P,Pj. Once such example is a removal of an obstruction in a region of interest in a system of conduits. In the medical realm, such a system of conduits may be a system of vessels of the human or mammal heart, or of the brain, or of other anatomies or organs. However, other medical applications, and even non-medical applications, are not excluded herein. But having said that, the following will mainly focus on image guided procedures in medical applications.

Broadly, the medical imaging arrangement MIA may include an imaging apparatus IA operable to acquire, for example, still imagery, or a stream of fluoroscopic or angiographic imagery, comprising frames acquired over time of the relevant anatomy of the patient, such as a region of interest (ROI) in respect of which the procedure is performed. Thus, the ROI may include location of an obstruction and its surroundings, including branches of the vessel system, etc. Thus, in one among many different scenarios, the imaging arrangement AR may facilitate obstruction removal, to so restore (or at least increase) at the affected site flow in a blood vessel or other conduit system.

More specifically, such or other facilitation may be enabled by a computing-based facilitator system FS of the medical imaging arrangement MIA. The computing-based facilitator system FS is configured to efficiently handle a set of certain computation models. The computational models may help in coordinating efficient use of the one or more implements, possibly together with other supporting equipment E, Ej in the image guided procedure.

On still a very broad level, and as an initial characterization, the facilitator FS may process such imagery *m=mt* provided by the imager IA to compute, based on such processing, a result δ in relation to one or more of the implements. The facilitator FS may further provide certain feedback data *e*. Optionally, the feedback data *e* and/or the result δ, and indeed the imagery *m* itself, may be visualized in graphics display which may be displayed on a display device DD under operation of a visualizer or graphics display generator VIZ. Visualizer VIZ may generate a graphics display which includes indication of any one, two or all of the feedback data *e*, the result δ, the imagery *m.* For example, the information *e* and/or δ may be combined with a current live image or stream as provided by the image apparatus IA.

The computerized facilitator system FS may be arranged in hardware or software, or partly in both. facilitator system FS may be integrated into an operator console OC of the imaging apparatus IA, or may be otherwise integrated in the machinery of the imaging apparatus or associated therewith. In other embodiments, the facilitator system FS may be implemented on one or more servers or other computing devices (e.g., desktop computer), located remote from the imaging apparatus, or located in the exam room with the imager IA, but in a separate housing. The facilitator system FS may be partly run on such a server and/or in addition or instead on a mobile computing device such as a laptop, smartphone or tablet, or any other terminal user device. Whichever one or more computing devices may be used, such computing devices may include one or more data processor circuitry (chip), such as CPU, GPU or any other, and storage media for storing code instructions. Such code instructions so stored may be capable of causing executing the functions of facilitator FS described above and herein below. Distributed computing, or cloud-computing may be used, if required. Some or more of the functions may be performed by one single computing device or multiple such computing devices, may be used, as required and apt in the circumstances.

Before explaining operation of the facilitator system FS (also referred to herein in brief as the "facilitator FS") in more detail, continued reference to Fig. 1 is made first to aspects of the imaging apparatus IA as such, in order to provide context which may assist such explanation.

The imaging apparatus IA ("imager" in brief), is preferably configured for fluoroscopy or angiographic imaging as mentioned above, for interventional imaging, although application in diagnostic purpose is not excluded herein. The imaging apparatus IA ("imager") is preferably X-ray based. The imager IA may be of the C-arm/U-arm type so may allow collection of projection raw data λ' along different projection directions P(α) around the region of interest, such as around the mentioned stricture/obstruction in a coronary vessel. However, non-rational radiography equipment is not excluded herein.

The imaging apparatus IA includes an X-ray source XS and an X-ray sensitive detector XD. In some embodiments, the X-ray source XS, and opposite thereto, the X-ray sensitive detector XD, are arranged preferably on a rotatable gantry GT. The gantry GT may have a recess in which an examination region is defined where the patient, or in particular the region of interest, resides during imaging. The rotatable gantry may thus have a "C", "U" or similar shape, such as in C-arm imaging apparatus envisaged herein. However, such or similar rotational or an otherwise dynamic setup, where there is at least some relative motion between source and detector intended, is not necessarily required herein in all embodiments. Instead, a planar radiographic setup is also envisaged in some embodiments. Instead of the rotatable setup as illustrated in Fig. 1, a bi-planar imaging setup with two detectors with intersecting imaging axes (for example at right angles) may be used. The recess shaped gantry may allow access of the interventionalist to operate one or more tools or devices, referred to herein as implements Pj, in order to perform intervention, such as angioplasty, obstruction removal at heart or brain, implantation of heart valve, or many others, not necessarily confined to blood vessel system, but may be sued instead of intervention in the urethral system, kidneys, abdomen, etc., More details in on such implements and their use in the present setup will be described later below in more detail.

In addition, whilst digital-at-source imaging, such as flat panel detectors is preferred, where the imagery is natively acquired as digital data, such as setup is not necessarily required. More traditional, cassette-operable, analog imaging methods (based on X-ray films) are not excluded herein. The analog imagery may be digitized post-acquisition for example, by using a digital camera. Imaging based on X-ray image intensifiers, etc. with post-digitization is also envisaged in some embodiments.

Generally, imaging includes energizing the X-ray source XS, so that an X-ray beam XB issues forth from a focal spot of the source XS, traverses the examination region (with the region of interest in it) and interacts with patient tissue matter. Interaction of X-ray beam XB with tissue matter causes the beam to be modified. The modified beam may then be detected at the detector XD. Conversion circuitry (not shown) converts detected intensities into digital imagery, in particular into projection imagery. The imagery may be used by user in the medical procedure. The patient may reside on a patient support PS such as a table during imaging. Soft tissue such as the one making up the coronary vessels, provide usually poor contrast in native X-ray imagery.

Optionally and according to some protocols, in order to boost contrast, a contrast agent (sometimes "CA" or short), such as an iodine solution, gadolinium, or other, is delivered at least at times through an access point AX into the blood flow of the patient prior to or during imaging. The contrast agent CA may be delivered via a CA delivery apparatus (not shown), such as a motorized pump, but manual delivery is not excluded herein. The delivery apparatus may be operable to deliver a defined volume of contrast agent to the patient. The volume of contrast agent so delivered travels with the blood flow. After a certain period of time contrast agent accumulates at the region of interest such as in or around the stricture. Image acquisition is preferably synchronized with arrival of contrast agent at the ROI. Preferably "contrasted" imagery is acquired whilst there is contrast agent CA present at the ROI. Optionally, additional "non-contrasted" imagery is acquired whilst no contrast agent is present at the ROI.

X-ray imaging is generally based on the attenuation co-efficient of the matter(s) that makes up the region of interest. The attenuation (coefficient) is dependent on the energy of the X-ray beam. In order to harness this energy dependency, a spectral imager IA may be used, configured for spectral imaging, but this optional. This may include detector-sided or source-sided solutions. Detector-sided solutions include photon-counting detector hardware or a dual-energy setup with dual-layer hardware for example. More than two detector layers may be used although this is less common. Source-side solutions include multi-source setups, kVp switching, filtration and other.

The imaging set-up as envisaged herein is however primarily intended for imagery as provided by a conventional (non-spectral) X-ray imaging set-up, the latter spectral imaging being merely an option herein and is as such not being excluded herein.

If spectral imaging is used, such as for more accurate visualization of material focused contrast, such as contrast agent (if used, which, is again, only optional herein), a spectral computational set-up may be used in some embodiment. Such a spectral imaging setup includes a spectral image processor SP. The spectral image processor SP may implement a spectral image processing algorithm, such as material decomposition or other in order to convert energy integrated projection image data λ into spectral data λ'.

For simplicity, the designation "*m*" may be used herein to indicate input imagery in general to be processed by the facilitator system FS. That is, "m" may relate to original projection imagery λ as acquired at detector XD or to spectral projection imagery λ' as computed by the spectral processor SP based on the original projection imagery as acquired. An imaging interface IIF provides this input imagery *m* for processing by facilitator FS in a wired and/or wireless manner over a suitable data communication infrastructure COM.

Imaging as envisaged herein is preferably attenuation-based, but this is not at the exclusion of other X-ray modalities, such as dark-field imaging and/or phase contrast imaging. Tomosynthetic or tomographic imaging is not excluded herein, due to the multi-directional imaging capability of the preferably rotational imaging apparatus IA. However, processing of projection imagery in projection domain is mainly envisaged herein in embodiments.

Broadly, but in more detail, what is proposed herein is a computing system CS, in particular a facilitator system FS, that facilities an image guided ("IG") procedure ("IGP"). More specifically, it provides computational support to such image guided procedure IGP. Image guided procedure may include in the medical field, image guided therapy ("IGT"), or similar for diagnosis or planning, or for any other medical objective. In general, in such procedures, imagery is used to guide the procedure or otherwise support the same. The IGP is in respect of a region of interest ("ROI") R.

To this end, the imaging apparatus IA is configured for dynamical adjustment of its imaging geometry to so obtain imagery *m* at different field of views ("FOV") of the region of interest R, "ROI", along different spatial directions, widths, and/or magnification, etc. In particular, adjusting such imaging geometry may include operating imager IA's actuators AC of any kind so that position and/or orientation of an imaginary imaging line can be adjusted in space. Such line may be thought to run from a focal spot of the imager's x-ray source XS to a center point of a radiation sensitive surface of the x-ray detector XD. Such line is changed in position and/or orientation in surrounding 3D space in which the subject, such as at least a part of the patient PAT, and by extension the region of interest R, resides. Whilst still imagery is not necessarily excluded herein, the imager IA is preferably operable to provide a time series of frames (images) *mt=m* that represent a live action video feed. The feed or stream of frames *mt* may be comprised of 2D frames of the fluoroscopic kind, or of the angiographic kind (angiograms), the latter being contrast enhanced by prior administration of contrast agent into the ROI R to so confer image contrast to natively radiation transparent structures, such as vessels. In addition, or instead to such projection domain imagery, reconstructed tomographic 3D imagery in image domain may be so provided by imager IA.

Medical IG procedures that may be supported by the proposed set-up may include angioplasty, thrombectomy, or cerebral interventions, urethral, abdominal, as needed Under guidance of a stream of frames *m*=*mt*, tools or devices referred to herein as implements P, Pj(j>=1), may be used, possibly in addition to other equipment E, Ek (k>=1). Such equipment may part or components of the imaging apparatus IA, or may be separate therefrom, such as a robot, a contrast agent delivery apparatus (such as a pump). Referring now in more detail to the mentioned one or more implements P,Pj, such may be used by robot or human user (interventional radiologist, or other) in the FOV. Thus, in the IGP, one or more such implements Pj, P may be within the field of view and are ascertainable as representations thereof in the acquired imagery *m.* The dynamic nature of the proposed IG set-up thus allows to visualize, in the live action video feed, motion (including deformation) of the implements as residing in the field of view, and as they are used in the procedure, as well as visualization of some or all of the surrounding anatomy. Adding a further layer of complexity, the surrounding anatomy may be likewise subject to motion, as may be caused by physiological activity, such a respiratory or cardiac activity, depending on the region of interest. For example, in a cardiac intervention and imaging thereof, the myocardium enmeshed by its vessel tree, with some of the implements residing in the vessel tree, are all subject to motion caused by the myocardium as its cycles through its cardiac states (systole and diastole), in addition to the motion of some of the implements, such as guidewires, catheter, flow diverter, stents, etc., as they are used during the procedure.

Cardiac interventions may include using such implements to treat a stenosis ( a pathologically structured vessel), remove an embolus/thrombus, or other flow occluding body, repairing a heart valve, implanting a new heart valve, and many other procedures. To the extent that these can be said invasive, they are minimally invasive.

What is proposed herein is the facilitation by facilitator FS of using computational models, in particular machine learning based, that, in concert with the image feed *mt,* are used to support the intervention, such by image-based object detection, in particular tracking such objects over time, segmentation, and so forth. In particular, as will be described below, a respective specialist computational model can be selected from a set or bank of models configured for different detection specialties, to so provide targeted, tailored, support for different phases of the procedure. For example, and specifically, one model so selected for detection of one specific one of the implements from plural implements, or of a particular part thereof relevant in given phase of the procedure. The selected computational model may then operate to detect and time track, over plural frames, in-frame/image objects that correspond to the said implement or part currently of relevance, until a different phase is entered into, at which point a different model is selected, and so forth. The tracked object locations as found by such computational models can be supplied to other supporting equipment E,Ej, such as a robot, or can be used to control one or more components of the imaging apparatus in order for them to perform a sub-procedure/task in given phase of the overall procedure. In this manner, computational power, and ultimately electrical energy, can be saved by selecting a specialist target model for the detection task, instead of using generalist models that may be too expensive to operate in terms of energy consumption. An addition, as opposed to selected specialist models, generalist model may not have the requisite robustness, may not be able to produce detection results at requisite level of confidence as may be needed for some of the proposed procedures at which higher precision may be called for.

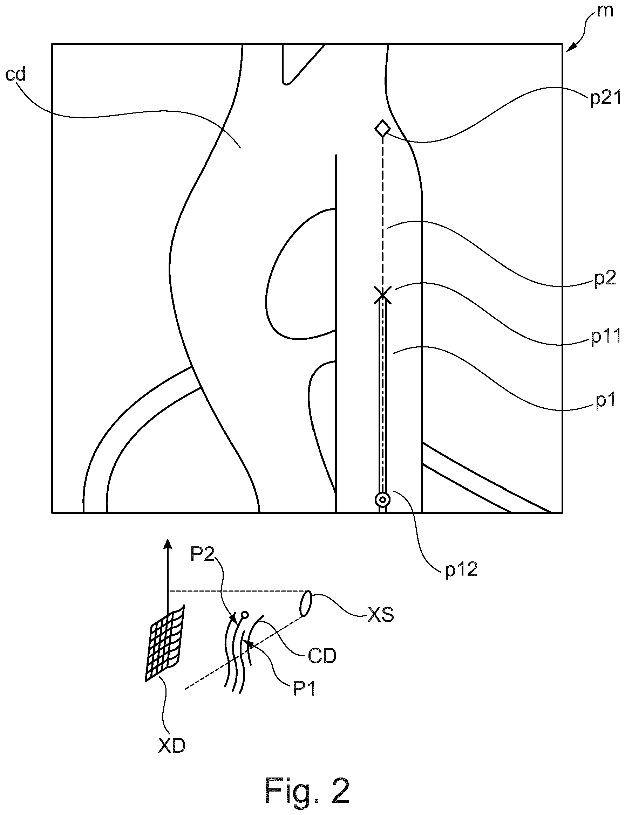

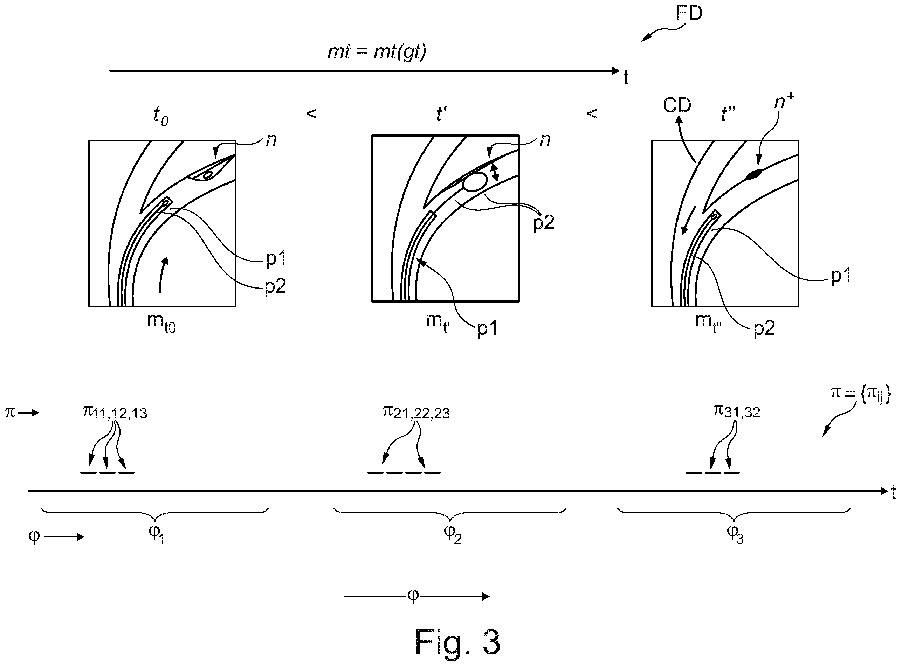

Before providing more details on the operation of the facilitator system FS as envisaged herein reference is made first to Figs 2 and 3, in order to illustrate some aspects surrounding image guided procedures as envisaged herein.

Referring first to Fig. 2 in more detail, this shows schematically an example illustration of a frame *m* as may be captured or acquired by the imaging apparatus during IG procedure. Implements P1,P2, such as guidewire(s), catheter(s), sheath(s) etc., may be used in the procedure and may reside in the field of view of the imaging apparatus within the patient and hence the examination region as is shown in the lower part of Fig. 2. This lower part of Fig. 2 shows a clipping of the 3D imaging examination region. The implements may reside in a system of conduits C, such as a blood vessel tree, which may comprise a system of branched arteries or veins, such as in the neck patient, or as part of the coronaries that enmesh the human myocardium, as may appertain to cardiac imaging and related IG procedures.

A projection representation of the 3D scene at the bottom of Fig. 2 is shown in the upper part of Fig. 2 in the form of the said projection frame *m,* with various in-image objects *pj, cd* that represent implements *Pj* or anatomy CD. Whilst the below will be confined in the main to projection domain imaging, this is not at the exclusion of reconstructed 3D image volumes, to which the principles of operation as described herein may be equally applied, and such is indeed envisaged herein in embodiments. Reference characters, in the upper portion of Fig. 2 relate to respective in-image representations/objects of the implements Pj and anatomies CD in the lower portion of Fig. 2. In general, lower case letters are used as reference signs herein for in-imaging representations/objects, whilst upper case letters are used to refer to material objects in 3D as such. Illustrated are projection footprints *cd* of the conduit system CD, and, in this example, two implements P2 and P1 with their footprints *p1,p2* and footprints of their related parts *p11, p12, p21.*

Implement P2 may be a guide wire having different components, portions or parts, and the like, such as its tip whose footprint *p21* is represented, or a footprint or any other, for the current phase, relevant section/point along its length, or attached functional add-on, or other. Another implement may be a sheath or catheter P1, a tubular structure, sliding over guidewire P2, with frame *m* representing footprint *p21* of its own tip, or other part or section such as a fringe portion P12 that is just visible in the current frame at the lower fringe. Thus, implements Pj as used herein, may be "nested", one or more in another, over one or more levels, as needed. The representations *p1,p2* of those implements P1, P2 and its parts or sub-components *pij* is merely for illustration. Other tools Pj>2 may be used instead, or in addition, to the ones illustrated in Fig. 2. Indeed, as mainly envisaged herein, multiple (two three or even more) tools may be in the field of view, with different parts and/or different states being relevant for use in different phases of the procedure. Sometimes, one or more implements are present, whilst one or more others are added or withdrawn from the current FOV. State changes of one or more implements may be recorded in frames mt, such as deformation of a ballon catheter, etc. Thus, the FOV may at times become "crowded", possibly with switching between different FOVs, which may be difficult for user to manage . In particular, novice users, may face a possibly confusing situation, with a wide array of different implements Pj being resident in the field of view, as situation further compounded when, as is often the case in IGP, the spatial FOV is adjusted possibly many times over. It is in particular management of such situations which can be facilitated by the facilitating system FS: whenever needed, in particular when coming out of a current phase and entering a different phase of the procedure, system FS may then select an appropriate supporting computational model M which helps "pick up" the implement or part thereof needed in that phase. Without such object detection help, user may not be able to easily tell which image representation (e.g., projection view (footprint) represents which implement P, or which part thereof, etc. But even for seasoned users, facilitator FS may lend a welcome hand in such situations as it can help combat user fatigue. For example, user may be stressed already on account of having to deal with a difficult case at hand or juggling a sizable workload, etc. Also, and more fundamentally, without the device or device part specific detection the associated automation task that supports the user in the procedure based on the detection, may not function correctly.

Referring now to Fig. 3, this is an illustration of a video feed FD comprised of time series of consecutive frames *mt* over times *t0, t', t"*, some or each possibly acquired at different geometry setting *gt*, that may confer a different FOV on the region of interest, and on one or more implements and/or their parts that may be resident, at least at times *t* in FOV. The times *t0, t*', *t"* in Fig. 2 may represent time instant or respective periods. Such change in FOV may not necessarily be caused by adjustments in imaging geometry as such, but is rather brought about by motion of one or more of the imaged implements Pj. As before, in Fig. 2, the frames *mt* may represent projection footprints *cd* of a conduit system CD, such as of a part of a blocked vessel, possibly branched, through which catheter P1 is navigated over guide wire P2 at time t0 to a lesioned site. At a later time *t',* on arrival at lesioned site *n,* one or more of the relevant medical implements is deployed such as balloon tip of balloon catheter P1 in an angioplasty procedure. As shown in such live action feed FD, balloon tip of catheter P1 emerges out of its surrounding transport catheter or P2, in order to expand and to treat lesion such as stenosis as may be visible at times as representation *n.* Once remedied, yielding mitigated stenosis feature *n+,* one or more of the tools P1, P2, are withdrawn at time t'. The upper portion of Fig. 3 illustrates the live action video feed mt captured during a procedure, in particular its dynamics, more particularly, the implements P1, P2 involved in performing the task, such as in this case angioplasty, or any other.

The lower part of Fig. 3 is a schematic representation of the procedure π comprised of sub-procedures *πᵢⱼ.* These may be thought to proceed in different consecutive phases, φ1, 2, 3 for example. There may be less than three or more such phases, the Figure being a mere schematic illustration. In some or each such phase φj, respective sub-procedures *πᵢⱼ* may be performed. Again, lower part of Fig. 3 is merely an illustration on another level as the procedure may be structurally more complex: thus, it may not necessarily have the linear structure as suggested by Fig. 3 (although it may well have). For example, some or all phases φj may overlap, etc., or may have a branching structure depending on the protocol envisaged. Some or each of the sub-procedures πin may involve the use of supportive equipment Eₖ that is operable to support the respective phase or sub-procedure, or any other task (such as an automatable task) performable via such equipment Ek in a given phase and/or procedure. Such equipment (devices, systems, etc.) may include parts or component of the imaging apparatus itself, for example its collimator COL (see Fig. 1), or other component that allow adjusting imaging geometry for example, based on the implement tracked by the selected model. In addition, or instead, equipment may include outside (ex-imager) equipment, such as surgical robot that may aid a user in performing the procedure. Some or each such sub-procedure may operate in a different context and may be reliant on a precise spatial detection and tracking over time of a specific one of the plural implements and/or a specific part of that implement, with other such implement or parts of the same implement being relevant for performing the different-sub procedures. In the illustrated feed FD in upper part of Figure, the phases /sub-procedure corresponded a navigation phase, deployment phase and withdrawal phase, but there may be phases of other nature, reliant on other type of implements or on parts thereof.

The facilitator system FS as envisaged herein is configured to provide such support through improved automated model selection. That is, the so selected model, selected from a bank of computational models, is likely to perform better than other such models from the bank in the required detection task of a specific one of the various implements involved, at a pre-determined level of quality in terms of robustness, accuracy and at requisite detection confidence. Different one or more pieces of equipment, different implements, or different part(s) of a given implement(s), may be relevant, at different times during procedure, in particular during the sub-procedures. Thus, such relevance may be a function of time, phase or sub-procedure, and/or the intended automation task that is based on the detection. Such automation task may involve control of a robot, or collimator, tube, detector, actuator, etc., such implement or part thereof may be said herein to be *"relevant"* for phase or sub-procedure, if such implement or part thereof is used/is operational to perform the respective sub-procedure. A similar language may be used herein, by extension, to the phase or sub-procedure being relevant. At times, a single implement may be in the FOV, but this may move, or different parts of it may be relevant for different sub-procedures.

The procedure II may be one of angioplasty as illustrated, with or without planting a stent (in the latter case the stent being another implement Pj), or procedure may be other, such as placing a flow diverter, or handling of an occlusion in the conduit system, such of a thrombus. Such handling may include multiple implements Pj, for example, one for dislodging the occluding mass (e.g., an aspirator), and then using another implement, such as an embolus retriever for evacuation out of conduit system, etc. Similar scenarios with multiple tools Pj use over multiple phases may be encountered ex-medical, such as in endoscopic inspection and repair or defects, such as in machines, engines, etc., or in plumbing, pipeline maintenance in Oil & Gas, or in numerous other fields of endeavor.

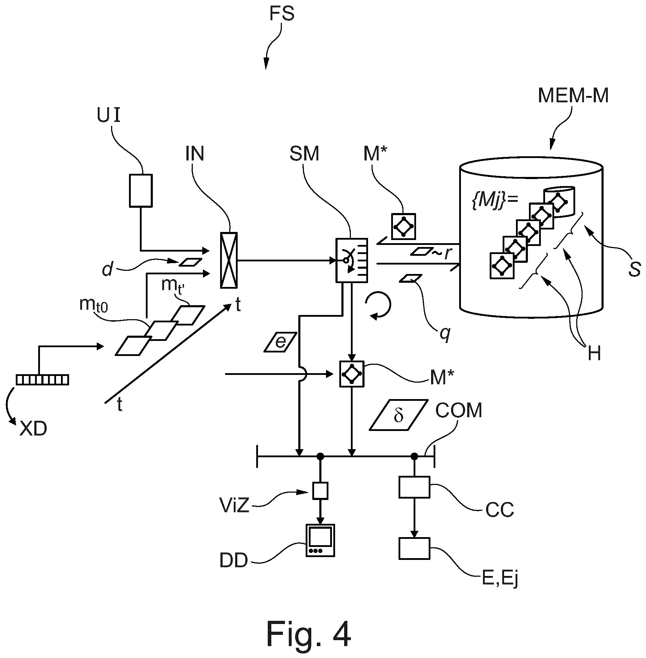

Operation of the facilitator system FS is now described in more detail with reference to Fig. 4. In operation, at one or more input ports IN of facilitator FS, input data *d* is received. The input data *d* may be based on one or more frames, such as the current frame *mt0*, receivable at the detector XD during image guided operation of the imager IA. Specially, the user may operate a user interface UI, such as a graphical user interface, to indicate a location in the current frame *mt0* only, and/or in one or more prior frames *mt, t<t0.* As option, in addition or instead of such user indicated in-image location, the whole of the frame is received at the input IN for processing. Such input data, in particular the user indicated in-image location, may be referred to herein as selection initiation data, as it initiates mode selection by a selector module SM. Other kinds of such selection initiation data may be provided, instead of, or in addition to such in-image location. The input data, in particular the in-image location, may be provided by user, or may be supplied automatically by the imaging apparatus or some computing node. The selection initiation data may include contextual data that describes, indicates or otherwise pertain to the imaging purpose, the current phase of the IGP generally, or to the current sub-procedure specially. In the mentioned example, the in-image location may be explicitly indicated by click action or other pointer action in the displayed image. However, the indication may instead be done implicitly, such as user selecting a specification of context, such as intended device (or a device part), and/or such as type, etc., or specification of intended phase, sub-procedure from a drop-down menu or by use of any other user interface. Thus, in such embodiment, the selection initiation data may include such specification, instead of, or instead, the explicitly indicated in-image location. Thus, in such case, the specification (input data) implicitly defines the in-image location. The facilitator FS could then select model M*, based on the specification (such as the automation task, the procedure phase, and the selected device, etc.). Thus, in such case the in-image location would be only implicitly indicated by the user. However, in the present disclosure may reference is made the explicit in-image selection, cognizant of the proviso that the said implicit selection via context data specification is equally envisaged herein in embodiments.

The in-image location of input data *d* may be provided in form of a bounding box, one or more in-image coordinates. The in-image location may indicate the in-image representation (projection or other "footprint") or the implement or part thereof on which the user may wish to focus the subsequent or ongoing sub-process in the current phase. Thus, the so indicated in-image location may pertain to the relevant implement or part thereof currently residing in the current FOV. This information D is then passed to a selector module SM. Selector module SM interface with a set *S* (pool, or bank) of computational models Mj that are held in a memory MEM-M. Specifically, non-volatile memory MEM-M may hold the set *S* of such models *S*= *{Mj}.* Memory MEM-M may be arranged as HBM (high band width memory) to keep access latencies at bay.

The models Mj may be arranged, processed, stored in a data structure H. Such data structure H may support models' Mj efficient identification and ascertaining of their aptness to the current detection task, and hence selection of suitable, such as the best, model M*. The current detection task is one for in-image detection of the relevant implement or part thereof to which the input data *D* pertains. Data structure H may be arranged as a relational database structure, a tree structure, an associated array structure, or any other may be used. Meta data µ =µj may be used to construct the data structure *H:* µj -> Mj. Meta data may serve as a key data for rapid lookup. The metadata may pertain to specification of some or each of the models Mj. Meta data may encode model specification, characteristics, and the like, for example in terms of their performance, provenance, or what type, class, or category of implements of parts thereof they have been trained on to detect, etc. However, use of such data structure H and/or or such meta data is optional. In particular, operation of selector module SM is not necessarily reliant on either, structure H or metadata.

The selector module SM may apply a selection policy to select an appropriate model M*, also referred to herein as target model. It may be the best such model as based on performance threshold(s), but that may not necessarily be needed in all cases, so long as it is established that selected model M* complies with certain performance threshold(s) as may be prescribed in selection policy. On selection, that is, once a selection test as defined in said policy is passed, the model M* may be selected. The facilitator FS may supply, on such selection of model M*, output data at output port OUT. Such output part may be indicative of the selection, such as may include an identification or handle for the selected model, may include model M*'s characteristic(s), or may include feedback data for user on the successful selection. The feedback data may include evidentiary data to prove to user appropriateness of selection, etc.

On selection, the selected model M* may then be retrieved, transferred, and/or loaded into a faster memory, or any other, to so have model M* ready for deployment to analyze (e.g., detect) frames for relevant implement(s). However, such transmitting, loading into other memory, etc., may not be needed necessarily, as the selection result may merely serve to identify the model M*, in whichever memory it is held. Thus, the selected model may remain in the current memory, and is "worked" (used). In either case, when model M* is worked/used, it is automatically applied to some or each follow up frame *mt*,t>0, to supply, in the ongoing phase of the procedure, sub-procedure, etc., the sought after detection result data *δ(mt)* , whenever needed, such as when a new frame *mt* is acquired, etc. The result data δ may then be visualized by a visualizer VIZ on the current or another display device DD, such result may be stored or otherwise processed. Thus, user need to provide their location indication (the selection initiation data) only once, e.g. when a new phase or sub procedure is entered into. From that point on, some or all subsequent frames are provided as input d' automatically, preferably as whole, and are processed, as they frames come in, by the currently selected model M* to detect the implement or part thereof in the stream of frames *mt, t>t0.* This can go on until user provides new input data d', at which point the above is repeated, that is, a new model is selected, and this is then used, and so on. The user can provide new input data (e.g., selection initiation data, such as in-image location/region indication for a new location) at any point, but is expected to most likely do this when the procedure enters into a new phase φj->φj+1, at which point another implement or part thereof, or another part of the current implement gains relevance. Preferably, as envisage herein, the selector module SM operates a "one-model-only" selection mode, in which, on selection of a new model in response to newly indicated in-image location in a frame, the current model is deselected, on selection of a new model M*', and so forth. Thus, in this mode, there is, at one given time, only a single model "active" to supply detection results. This ensures targeted support at low computational spend. However, at times, a multi-model mode may be preferred, and such is indeed envisaged, where more than one target models can be selected and remain active at a given time, each detecting for different implements or parts. Thus, in this mode, multiple detection results, at least one from each model, may be supplied to support parallel sub-procedures, such as controlling two items of support equipment's Ek, Ek', as the case may be. User interface UI may include an option to dynamically switch between these modes in a given procedure Π.

In any of the above mentioned embodiments, modes. Etc., the computational result δ, preferably the implement detection result, as output by the model during its used in phase/sub-procedure once selected, may be forwarded through the communication infrastructure COM. For example, the output data δ may be passed to control circuitry CC. Based on the detection data, the control circuitry allows controlling one or more of the supporting equipment Ek,Ek' that operate to produce their output or perform a task thus likewise supporting the ongoing phase as intended. Because the imaging geometry as was used to acquired the frame in which the selected model M* detected the relevant implement Pi or part Pij thereof, such detected implement can localized in space by a localizer module (not shown). Such module may be part of the control circuitry CC, or may be performed by a an intermediate node, interstaged between selected mode M* and control circuitry CC. This is at least because some such equipment may require, for their operation, spatial knowledge/data on the whereabouts in space of the in-image detected implement. For example, some such equipment Eₖ may include collimator COL, and this controlled such that collimation window follows the relevant implement during motion to so acquire collimated frames to save dose. Similar for a robot, that may assist user in performing the intervention. Arm, effector, etc. or other components of robot E_{k'} may be controlled by control circuitry CC, based on the image-based tracking of the relevant implement. Other equipment so controlled may include a contrast agent (a dye) delivery apparatus (e.g., a pump) that is controlled, based on δ, to release contrast agent at a clinically apt moment, to so boost image contrast when and where it is needed.

The various input/output data items explained above in relation to Fig. 4 and their use by related computational entities or nodes of facilitator FS are now described in yet more detail.

To this end, turning now first, again, but in more detail, to the input data *d,* this may take (as briefly alluded above) the form of a bounding box, a single (in-image) co-ordinate, or a group of co-ordinates that specify in-image location of a relevant implement's footprint in the current image only. The term *"location"* as used herein in this connection is meant to include a single point location but also a multipoint location (a 2D region that is). The indicated location as per input data *d* may be user-supplied, in whichever form, through a GUI or other. For example, a single or multi-click by a pointer tool, touch screen action, or stylus action, or any other, gesture based input or not, may be used. Other input options include gaze detection technology such as via a headset or glass, or other wearables or non-wearables, may be used to indicate the selection initiation data, of which the in-image location is one example. In some such technologies, eyeball movement is analyzed, and is then, based such analysis, translated into the in-image location of relevance for the current phase or sub-procedure. In addition, speech recognition may be used. Optionally, in particular in click or touch or other gesture action, the so user-indicated location is auto-expanded into a region, an in-image neighborhood, of sufficient size to ensure the local image data therein carries enough image structure to ensure meaningful input that can be acted on by selector module SM ("selector") with sufficient robustness for model selection. The selection initiation data may also include, or may include instead of any of the above, specification of a device type preselection from a drop down menu or other, which is then automatically located in the image, which forms an implicit kind of user indicated location.

Image entropy measures, image value distribution variation measures, etc. may be used to drive this expansion. In some embodiments, but not all embodiments, even when input *d* includes such user indicated in-image/frame location, or where such location is indicated by some automated functionality, it is still the whole frame that is passed on as input to the selector SM, together with the indicated input. Thus, in such embodiment the input data is of multi-channel nature.

The input data *d,* by way of the location indication, at least implicitly specifies the current or upcoming sub-procedure or phase φ in the ongoing image procedure. As said, the selection initiation data may be in form of a click or other user vs image interaction, and/or may include instead, or in addition, another signal indicative of the current phase /sub-phase. For example, user may indicate specifically, by text, speech or any other, directly an identifier of the relevant sub-procedure, and this information is used in conjunction with the image information (the whole frame and/or the indicated in-image location). Again, in such embodiments, case the selector SM configured to expect multi-channel input data d, in which the input data so collected does not only include image information but also contextual data of any kind, that pertains to the relevant phase or sub-procedure, such as meta data drawn from a header data of the frames. Later, once model is selected, the contextual data may be passed on to the model M* for processing, to so provide data-based regularization for better boosted performance.

As mentioned, the selector SM may use a suitable selection policy to make the selection of the appropriate target model M* based on the input data d. Different options for such selection may be envisaged herein in different embodiments of selector SM.

According to one class of such selection policies, and as per some embodiments, a polling or sampling is run by providing request *q* to the models and polling their respective "sample" responses *r,* which are then analyzed according to policy in order to make the selection. Such responses may include the detection result of the model M* based on query q. Query q may be based on the input data d. Detection results provided by the (candidate) models during operation of the selector SM in applying the policy are referred to herein as test detection (result) *r,* as opposed to the detection results δ the target model is producing post-selection. Such policy may prescribe a test protocol to be run on the models in the bank S, in turn or in parallel, to establish the target model M*. That is, once such sample response *q* indicates that is test passed, this may be used as feedback to user, as evidentiary information that the selected model M* is apt. The sample query *q* that is run past plural, in particular all models, or in a category of models, may include some or all of the input data. The model category may be determined based on the structuring H of the set *S* of models. In particular, the user indicated in-image location, optionally with the whole of the current frame, are passed as query against model. The model's response is thus taken as a test detection, and this is analyzed. Only when the model is selected (and hence trusted) can it be used to detect the relevant implement fully automatically in subsequent frames as detection data δ, preferably without user input, until user issues new input *d'* which may cause deselection of current target model, and selection of another model instead, or a further target model is so selected, depending on the mode used as mentioned above.

As per the selection policy, the model that responds appropriately is selected. Such appropriateness may be judged by pre-defined criteria as per the test to be used. For example, some models, of the regression or classifier type, may have been trained to return a bounding box δ that includes the attempted detection result as response *q,* in response to query *q* based on input data *d.* Such response of the model tested may include natively a confidence measure, or such measure may be derived by invoking a query tool that analyses weights or other parameters of the tested candidate model. Specifically, the query *q* may generally include the input data *d* as supplied by the user, thus may so include in particular the in-image location of interest. In the current test context, as opposed to use of model post selection, the in-image location forms an example of the selection initiation data, now used to initiate the selection operation. This location may generally include or designate a portion of the projection or image footprint in respect of the relevant implement Pj or parts thereof Pij as judged by user.

In embodiments, the selector SM may use one or more of a range of different metrics to measure performance of (candidate) model in terms of their response, and selection is based on a test in which the measure is evaluated. For example, in one embodiment, the selector SM on receipt of the sample detection response *r* may threshold the associated confidence measure, based on which thresholding the mode is selected, or rejected, and selector then moves on to next model in the set S, and so forth until a model is found that meets the threshold, and this model is then selected. For example, the confidence measure may include a percentage value of how confident the model is in having detected the in-image structure based on query *q* (and hence on *d*). If this percentage value, meets or exceeds the threshold, such as 95%, the model is selected. This confidence measure-based selection policy may yield plural models. In such a case, a selection menu may be displayed on the screen for the user to finalize selection, or otherwise a random selection is made by random number generation as needed. Alternatively, and as said, a first to pass selection is used.

In some embodiments, for improved responsiveness for the selection procedure, the classification structure H in which the models Mj of set S are stored is harnessed. For example, models may be ordered and grouped according to their specification or characteristics. For example, the characteristics may include training details of the respective model. Specifically, the characteristics may include specifications of the implements that the respective model was trained to detect. These may be grouped according to functionality, or similarity, such as clinical use, etc. For example, the implements in question may include types of catheters or guide wires that are distinguished by material type of which they are formed, the shape they are capable of assuming, or their purpose. For example, the following catheter groups may be used to store and structure such specifications in a stratified manner. For example, the specifications may specify whether the catheter in question is vascular or non-vascular, it may specify that it is for guidance or diagnostic or for selective or for flushing as needed. The specification may further include diameter or gauge size, such as *0.018"* (inch), *0.035* ", etc. The specification may specify whether the implement has a hydrophilic or hydrophobic coating. The specification may indicate the stiffness. The specification may specify the intended functions such as guidewire, sheath, catheter, etc. The specification may specify tradename or other type, such as "Nitrex", "Cope", "New Yorker", "Rosen", "Roadrunner", etc., or any other specification. Such specifications may relate to tip length, its shape, the material of wire's core, its coating etc., overall length, etc. This structuring H based on specification of the respective implement, which the respective model was trained to detect, can be used to conduct a more efficient selection test. For example, if a certain model in a class as per the specification H has failed the test, all models in the same or similar classes can be ruled out, and the search/test can focus on other classes, and so forth.

Another selection policy may be based on explainability/interpretability concepts and related metric, as pertain in particular to ML models Mj, as mainly envisaged herein. See for example Ramprasaath R. Selvaraju et al in "Grad-CAM: Visual Explanations from Deep Networks via Gradient-based Localization", published in arXiv: 1610.02391 (2019*).* In this embodiment, as before, models are sampled based on test input q (the input data) to obtain test response per model Mj sampled. The test is now based on comparing set theoretic intersection (overlap) of set of pixels / voxels in current frame mt0. The two sets per model are the current image mt0 and the indicated in-image location (which in this embodiment is best thought of as a region in mt0), and an explainability region based on test output detection r. The explainability region is the set of all those portions in image mt0 that have contributed more than others to the test detection result r. Thus, the explainability region ⊂ mt0 measures which part of the input image *mt0* the model has "looked at" when coming up with output r. The larger intersection (overlap) between the two sets, the higher the likelihood that model is suitable. The overlap may be measured by a suitable metric, such as Dice coefficient, or Jacquard coefficient or other. Thresholding against such coefficient or other overlap size measure may be used to establish target M*. First to pass or run-off selection from candidates that have passed the test may be used. Again, if available, the data structure H may be used narrowing down the field of models which are actually polled for their responses r. But, as said, the query-response-(*q*,*r*) type embodiments are not reliant, as such, on such structure H.

Turning now in more detail to the data output e provided by the selector model SM, this may include a handle, indication other identification information of the selected target M* judged suitable, or even best, for current purposes such as detection/tracking. This identification information may be used to pass follow up frames to the target model M* to have it generate detections for tracking the relevant implement or part thereof across said follow up frames, until user issues new input data d', as mentioned earlier. Preferably, but not necessarily, the data output e may include feedback data, such as an explicit indication on the selected target model M*. For example, the feedback data in output data *e* may include evidential data that demonstrates to the user the suitability or appropriateness of the selected M*. For example, such feedback data may take the form of instructions for visualization. Such data feedback data may cause modulating a visualization of the current data, such as the currently displayed frame *mt0* based on which selection was made. In particular, bounding box or other visual indication, such as by color-coding, is provided to visually overlay the detection output *r* of that model M* for the current frame and indicate this on the current frame to the user. For example, a segmentation or bounding box of the detection r may be displayed, color coded in visual distention against background. Ideally, the overlay may largely conform in position and shape to the projection footprint of the relevant implement as shown in the current frame and has been indicated by user in their initial input. Thus, highlighting the test result *r* as overlay or other graphical modulation in current image provides to user a convenient cross-check. Such indication may be or may also be included for the detection results δ in one or more of the follow up frames. User may enable or disable this option by suitable user interface functionality. When used in multi-model mode, when there are multiple target model used, each current selection may be so highlighted one at time, whilst others are greyed-out, or are simply not highlighted. As another multi-model mode, whilst other implements are tracked/could be tracked, they are not highlighted, as opposed to the current, relevant implement whose footprint is highlighted differently, such as in a greyed-out overlay, or other, as opposed to colored (red, green, etc.) of the footprint of the currently relevant implement. Thus, in this or similar embodiments, the selector model may provide in its output data real-time, dynamic, evidential information to confirm selection is sound. If there is a mismatch, between user expectation and delivered detection, user can abort the support by facilitator FS or take other cautionary measures. In such cases, it may be that the models used by selector to select from are not of expected quality, thus, have been trained on unsuitable training data, or training was otherwise flawed. Models found to cause such mismatch may be flagged for retirement, or may be flagged-up for retraining, or may be otherwise made object of remedial action.

Turning briefly to machine learning as pertains to the case where the bank *S* of models Mj on which the selector SM acts, such may be formed of machine learning based models, earlier trained on training data and then held into memory, awaiting their selection and use. Such trained models may include, a particular architecture, learned parameters, and one or more algorithms or procedures for using such trained models (inference or deployment) and for training same based on training data. Such training results in the learned parameters. The models as held are assumed ML trained herein on suitable, likely different, data sets. Each model has been trained for in-image detection of a particular kind, type, calls or category of different implements. Whilst the models are preferably machine learning based, explicit analytical computational models are not excluded herein, such as snake or region growing -based segmentation, or shape model-based segmentation, or any other. However, machine learning models are preferred as they generalize best it has been found.

Confining the following now to ML, and in more detail, the machine learning models Mj have been trained using a suitable architecture, such as decision trees, support vector machines, or, preferably, ( (C)onvolutional) neural networks ("NN"), as such CNNs, have been found to be particularly useful for processing spatial data, such as is the fluor- or angio-frames *m=mt* at hand. Each frame can be processed on its own, separately as it is acquired, by feeding same into the selected model M* *∈* {Mj}, is then processed by the model according to the algorithm (e.g., forward propagation in NN type models) to obtain the result *δ=δ(mt) =M*(mt),* with *"M***()*" being a shorthand notion for the model * viewed as function that maps input mt to output δ (detection result). In other to account for the sequential nature of the stream mt, earlier frames may be also feed into the model for processing. In addition or instead, recurrent NNs may be used or attention mechanism based models.

The models in set/bank S have been trained on a respective corpus *C* of synthetically generated or historical training data, such as imagery which may be located in PACS or hospital information systems etc., and pertain to earlier imaging cases or vignettes of prior patients. Such training may be based on a training algorithms, such as gradient-based methods, such as back-propagation based, or any other. In general, training may be formulated as an optimization procedure to improve a cost function. The cost function is a function of the mismatch between training output and associated ground truth data targets. Each set of training data, for a given implement, includes training input data processable by model in training to obtain the training output, and the ground truth, in particular for supervised or semi-supervised training setups. Unsupervised or enforcement training arrangements are also envisaged. Training may be a one-off, or may be repeated as new training data becomes available. The training corpus *C* is a set of sets C={*cj*}, with each training data set cj pertaining to imagery that features footprint for the respective class, type, etc. of implements (GW, catheter). Each such set may have been annotated by human experts to indicate the footprint of interest. The unannotated version may be used as training input and the annotated version is the ground truth, which the respective mode Mj is trained to meet. In training, an initial set of model parameters are adapted, to improve the cost function. This may be done in various iterations until a stopping condition is met at which point the respective model is trained, and can be stored in the bank S. This procedure is repeated until all models Mj are trained. ML models and their training is covered elsewhere in more details, such as in A. Shrestha et al, "Review of Deep Learning Algorithms and Architectures," published IEEE Access, vol. 7, pp. 53040-53065 (2019).

Each training data set ci for a particular implement type, mode, make, specification, etc., should include a sufficient number of different images that show the respective model in sufficient array of variations in terms of anatomies, positions and configurations, including in nested configuration, as may often be encountered in clinical practice, such as with guidewire (GW) being inside catheter, or sheath, etc. Such variation should also account for imagery acquired at different imaging geometries (collimator settings, different position of source XS/detector XD/gantry) as may often happen in IGPs. Carefully annotation may be need by experts to distinguish implements in such nested configuration as per their combined footprint. A good mix of fluoroscopic frames and angiograms may be beneficial for improved learning. Training may be stratified further for each implement model, to so obtain further specialist models trained to detect a specific (and from model to model different) part or component of the same implement. Thus, hierarchy of trained models may be obtained, including respective "parent" models, each trained to the detect specific implement of a specific type, class, make, etc., Some or each such parent may be associated with one or more "child models", each trained for specialist detection of only a subpart /component (e.g., tip etc.) of the same implement, which implement the parent model was trained to detect as a whole. Of note, the said association of parent model and child model(s) is merely a logical one, as each such model, parent and any child, constitutes a completely differently trained model, trained to pick up different in-image features.

Models may include multiple data channels to accept context data, in addition to frame. Such context data that may indicate context, and this is then feed into model in conjunction with current frame *mt0*,*)* and is co-processed by model. Such contextual data may include patient data, bio-characteristics or data that indicates the phase or sub-procedure, etc., device specification, e.g. stiffness. For example, the specified stiffness may define a certain min-curvature, etc.

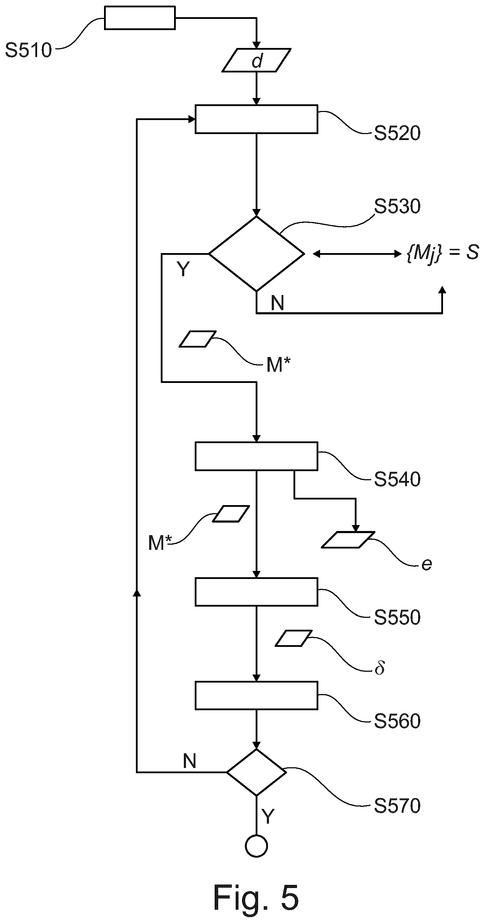

Reference is now made to Fig. 5 which shows a flow chart of a computing system-implemented method as may be used herein to implement the above facilitator system FS. However, it will be understood that the below aspects and steps are not necessarily confined to the architecture in above Figures, but may instead be construed as a teaching in their own right.

At step S520 input data *d* is received. This may be based on one or more image frames (images in a time series) acquired by projection, x-ray or any other imaging apparatus as acquired at step S5 10. The imaging apparatus is preferably of the interventional type. Some or all frames may be acquired at different imaging geometries. The input data may pertain the current frame mt0 as acquired. In some such frames, such as the current frame, one or more implements may be in the FOV and leave their in-image representations, such their projection footprints. The current frame may be displayed. The input data may include the whole frame and/or an indication of at least a part of the in-image representation of a relevant implement or a part/component thereof, relevant for a current phase or sub-procedure of the current ongoing procedure. The stream of frames are for image-guided support of such procedure. The indication may be provided interactively, by a user interface, such as via a GUI based on the currently displayed frame, by a pointer tool, by speech, gaze detection, or in any other way.

At step S530 a selection is then made based on the input data to select a computational model, such as a trained machine learning model, from a said pool of trained models available. A selection policy, possibly prescribing a selection test, may be applied to make the selection of target model from among plural models. Each model has been trained to for specialist detection of particular type, class, model make etc., of implement. Thus, the pool("bank") may include at least once such model, trained for respective implement type, class, etc., in-image detection. Each such type, class, etc., of implement may have different specifications, characteristics etc., such as shape, form, length, stiffness/min bending curvature, etc.

Appearance, structure, or other quality etc., of the respective in-image presentation in a projection image/frame for example may be a function such specification, characteristics of the respective implement. Some selection policies (called herein (*q,r*)-tests or similar) as used in selection step S530 may include applying the input data as query data *q* to the model and examining the respective responses/outputs r as per the policy/test to establish target model. Instead of, or in addition, meta-data of the stored models may be used for selection. Such meta-data may indicate the type of implements on which the respective model was trained. Input data (e.g. current and/or past frames) may be analyzed to establish type, class, category of the respective implement, and this is matched against the meta-data to establish target model. The use of the above mentioned query-response (*q,r*) type policies as mentioned above ( e.g., confidence based, explainability region-based, etc.) has the advantage that no such metadata is needed. Any pool of models from any vendors may be used, without prior "on-boarding" of model, other than loading some into the pool S.

Whilst the selection model may be based on repeated querying the models with response examination in the various r,1-setups described herein, a purely metadata based rule bases selection is not included herein, in particular when models are stored in a structure H based on such metadata. However, such a structure H may also be based in the query-response *(q,r)*-test, where the model may be used to more efficiently search candidate models to be queried, and which to leave out.

The input data may indicate an in-image location in the current frame and/or indicate the current procedure which the user wishes to have supported by certain equipment E, and for which control precise in-image detection of the implement or part thereof is required.

If the selection test does not (N) pass, the selection test is continued, or if it remains unsuccessful, an alert message is passed onto the user in whichever form. If the selection test is passed (Y), the so selected model one or more "target" models M* (as they may be now called once selected) are then made available for use as current target model(s) at step S540. The target model may now be used and is operable. For example, in one such use or target model deployment step S550, the target model may be fed with the current or subsequent frames for processing, in particular for detection and tracking of the relevant implement, or part thereof, as was specified in the initial input data S520 provided by the user or automatedly supplied through protocol.

In more detail, in said step S550, in subsequent one or more frames acquired after the current frame *mt0*, the selected model M* is deployed to produce a stream of detection results δ, at least one per frame in the stream of frames, so as to track the implement of interest for which detection the model was specially trained. Thus, in particular, the selection at step S530 yielded a model that has been trained on the same or similar type of implements for detection as had been identified at the input data by the user. Thus, at step S550 the model is applied to subsequent frames to support the sub-procedure in the current phase by detecting repeatedly, in the course of the frames, respective locations of the implement of interest whose position, orientation, shape etc. may change. After selection, there is no need for user to provide further in-image input as they did prior to selection at step S530, as the selected target model now operates automatically on a per frame base.

If the user does provide new input d' at step S520 in an ongoing deployment in a current IGP, this may trigger in embodiments a repeat of the above steps to select a new model M*', now specialized to detect a new implement or new part of the current implement in a new phase or sub-procedure, etc. Selection of such new model M*' may automatically de-select the current target, or multiple models remain active, as needed.

The models are generally trained so that they are invariant in respect to orientation and imaging geometry change. Particularly, the models have been so trained that they are capable of detecting the implement from whichever the field of view, as such field of view may be changing during the procedure and even during a current phase of the procedure, in particular in IPGs. Thus, the models have been trained to detect the respective implement model, type or class for which they have been trained for under different imaging geometries, and have been trained in a specialized fashion, to respond only to different types of implements, or specific parts thereof, as mentioned earlier. The selector module may further be trained to be able to differentiate the phases, sub-procedures. For this, in the training, the different (image) backgrounds over which the implements' footprint passes in the different phases, sub-procedures, etc., can be harnessed herein. Thus, the selection of training data is preferably ensured that the background is shown in the training imagery in sufficient variations and in different numbers such training image samples, so ensure that the model can map out the correlation pattern of phase/sub-procedure vs background.

At step S560, based on the detection result δ, the sub-procedure or procedure is then performed. This may include controlling a collimator, other equipment of the imager, controlling a contrast agent delivery device, a robot, or any other.

At step S570 it is established whether the procedure has come to an end. If not (N), the process repeats to step S520 to await new input data and the above repeats as often as necessary. If procedure terminated (Y), the process retires.

With further to earlier step S540 of providing access to the selected model by providing a suitable handle or identification or other, in deployment at S550, in addition, and optionally, output data e may include feedback (data) on the selection process S530 may be optionally provided to the user as further output data. Specifically, this feedback/(data) may indicate the response *r* of the model as elicited in the selection step S530, based on the current frame as received at input data d and as provide as sample query q to the (then candidate) model.

This feedback may include visually indicating by a bounding box or other highlighting, segmentation etc., the response *r* as used in the selection test, or as may be triggered even if a rule-based selection was made.

The response *r* provides a test detection as evidentiary information to user that selection is as expected and sound. For example, when the sample test result *r* is visually rendered in the current frame, this will ideally correspond to the intended user indication as indicated initially at input step *d,* via user interface pointer tool or whichever way.

The following refinements are also envisaged herein in some embodiments: the feedback information e on the selected model M* may later be used to add training data or vendor feedback (log files) if tracking was not accurate enough or not optimized for a new device.

The tracking data δ may be used to enhance the visibility of one or more of the implements.

The proposed setup can be used in platform for integration of trained models or tracking algorithms that are provided by third parties e.g. device manufacturers can provide an algorithm together with their devices.

It will be understood that that the method in Fig. 5 can be used in iterative refinement. For example, the selected model, once providing output may still detected two devices instead of the intended one, and/or multiple parts of such one or more devices. In this case, the multiple detections can be highlighted as overlays, and user can provide more refined, "2^{nd} generation", initial information by indicating in image or otherwise, the intended device or part among the detections, and this is feed as new, updated input into step S530 to re-select a new model that may be able to provide more focused, more targeted, detection for the device wanted. Thus, in this embodiment, step S550 when selected model is used in deployment (inference") and plural detections are mad and displayed, process flow may return to step S520 where new input data is received, in particular including an indication based on at least one (such as a single one) of the detected devices/parts and they marked up (highlighted, overlayed) footprints, to so restart selection at step S350 for a new model, and so on in more cycles, if need, with user providing later generation initial information.

Referring now back to embodiment when the input data S520 includes context data (implicit indication of in-image/frame location), in such case, the model may be selected using a rule-based approach for example. In such rule-based approach, the specification (such as a tag, code, etc.) is matched against identifiers H or similar structure that indexes the models as held in storage. If a match is found, the model is returned.

With general reference to the above, the following example application scenario may be envisaged herein, and is provided hereinunder as an illustration of the principles described herein: during an IG procedure that is assisted by an automated support function, (e.g. automated collimation, zooming, device enhancement, panning, robotic device manipulation, etc.), user provides an input as to which device, which devices, or which part of a device is relevant for a support function. This can be done by clicking on relevant locations on the devices that are currently visible in an image. It can be a drop down menu to select the specific devices that are currently used. It may be a region of interest drawn in the image. Even a verbal input is feasible using voice recognition. Or input information based on eye tracking. A metric is calculated for the various available neural networks to define which networks are most likely able to fulfill the indicated device tracking task. In the case of a user click on a device location the response function of all available networks is calculated and a predefined number of networks with the highest score, or above a threshold response value are selected. If a specific device is defined by the user, only networks trained for that specific device are selected. If the input is ambiguous, e.g. guide wire tip (without manufacturer, model, size, etc.) A selection process based on network response function in the image is added to the preselection of "guide wire networks". To make such an evaluation most efficient the neural networks are grouped based on similarity. I.e. if a stent retriever network for a specific type has a very low response function, other stent retriever networks for similar device do not need to be evaluated in the metric calculation. A feedback of the model selection indicates to the user what information is currently generated by the selected neural networks, how reliable this information is, and/or how it is used in the automated support function. This feedback may be an overlay of the response function of a network. It may be a displayed number of the tracking probability. Furthermore, the feedback indicates the responses from multiple selected networks (e.g. top 5) so that the user may easily refine his input after the first NN selection. E.g. the user clicks on an in-frame representation of, say, a flow diverter. One ML models then highlights the tip with, for example, a red overlay, one other the radio opaque markers with a green overlay, and a third network highlights the full flow diverter body in yellow, as one illustrative example. The user can then easily select which type of tracking was indicated by his first click, as per the refined/iterative mode as mentioned above. In another example, the network that works well on the first few frames may lose accuracy in later frames since it was not the optimal network for the tracking task. The user can then easily see if a better tracking option is available. Other feedback options may include one or more of: i) specification (population size, provenance, statistics information on feature distribution, etc.) of training data on which the current model was trained , ii) what device/function is this network trained for e.g. if a new device is used the user can recognize that there may be uncertain tracking since the new device is not on the training list of the selected network, iii) when was the network last updated, iv), in a multi-mode, other implements that are tracked/or that could be tracked but that are currently not used in the automated support function may be overlayed in grey.

The components of system FS may be implemented as one or more software modules, run on one or more general-purpose processing units PU such as a workstation associated with the imager IA, or on a server computer associated with a group of imagers. The workstation or other data processing unit(s) PU may be part of the imaging apparatus IA.

Alternatively, some or all components of system FS may be arranged in hardware such as a suitably programmed microcontroller or microprocessor, such an FPGA (field-programmable-gate-array) or as a hardwired IC chip, an application specific integrated circuitry (ASIC), integrated into the imaging system FS. In a further embodiment still, system FS may be implemented in both, partly in software and partly in hardware.

The different components of system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1. System (SS) for supporting a procedure in respect of an entity (PAT), the procedure guidable based on a stream of frames (*mj*) acquirable by an interventional imaging apparatus (IA) of the entity (PAT), in respect of which the procedure is performable via one or plural implements (P,Pj), at least one of which is residable, at least at times, in a field of view, FOV, of the imaging apparatus (IA), the system comprising:
an input interface (IN) for receiving, via a user interface (UI), input data (*d*) indicative of an intended one of the implements (Pi, P1,P2) in a current FOV as per a current frame *(mt0)* displayable by a display device (DID);
a selector module (SM) operable to select, based on the input data, a trained machine learning, ML, model (M*) from a set (*S*) of trained ML models, the selected ML model (M*) capable of supporting the procedure by being capable of providing a detection response, based on a current frames, of at least a part (Pij) of the at least one of the intended implements as per at least the current frame *(mt0),* and/or in at least one future frame (*mt*'); and
an output interface (OUT) for providing output data (*e*) including feedback data to a user on the selected model based on the said response.

2. System of claim 1, wherein the input data (*d*) is indicative of a task, the procedure, phase of the procedure, or sub-procedure, to be performed, and/or wherein ii) the input data (*d*) is indicative of an in-frame location of the intended one of the implements (Pi, P1,P2), and wherein the said task, procedure, phase of the procedure, or sub-procedure is supportable by one or more of the implements (Pi, P1, P2).

3. System of claim 1 or 2, wherein the user interface (UI) is a graphical user interface.

4. System of any one of the preceding claims, wherein selection operation by selector module (SM) is dynamic so as to change with FOV.

5. System of any one of the preceding claims, wherein selection operation by selector module (SM) is based on responses elicitable from the models in the set, based on the said input.

6. System of any one of the preceding claims, wherein the selector module (SM) is capable to so select based on evaluating a metric configured to measure respective responses of models from the set of said models.

7. System of any one of the preceding claims, wherein the output data (e) includes evidential information capable of indicating evidence on the appropriateness of the selection in supporting a task.

8. System of claim 7, wherein the said task includes causing controlling of equipment (Ej, E).

9. System of claim 7 or 8, wherein the said equipment (Ej,E) includes any one of more of: the imaging apparatus or a part thereof, a robot, and/or other one or more items of equipment usable to support the task.

10. System of claim 9, wherein the equipment includes a collimator (COL) of the imaging apparatus.

11. System of any one of the preceding claims, wherein the feedback includes providing information on the training of the selected model.

12. An arrangement (AR) for image guided support of a procedure, comprising the system (SS) of any one of the previous claims, and further comprising any one or more of: i) the imaging apparatus, ii) display device on which the frames are displayable, iii) at least one data storage or memory on which is storable at least one of the models that form the said set, iv) at least one of the implements (P,Pj), (v) at least one of the controllable equipment (E,Ej).

13. A computing implemented method for supporting a procedure in respect of an entity (PAT), the procedure guidable based on a stream of frames (*mj*) acquirable by an interventional imaging apparatus (IA) of the entity (PAT), in respect of which the procedure is performable via one or plural implements (P,Pj), at least one of which is residable, at least at times, in a field of view, FOV, of the imaging apparatus (IA), the method comprising :-
receiving, via a user interface (UI), input data (*d*) indicative of an intended one of the implements (Pi, P1,P2) in a current FOV as per a current frame *(mt0)* displayable by a display device (DID);
selecting, based on the input data, a trained machine learning, ML, model (M*) from a set (*S*) of trained ML models, the selected ML model (M*) capable of supporting the procedure by being capable of providing a detection response, based on a current frames, of at least a part (Pij) of the at least one of the intended implements as per at least the current frame (*mt0*), and in at least one future frame (*mt*'); and
providing output data (e) including feedback data to a user on the selected model based on the said response.

14. A computer program element, which, when being executed by at least one processing unit, is adapted to cause the processing unit to perform the method of claim 13.

15. At least one computer readable medium having stored thereon the program element of claim 14.
